(19) Europäisches Patentamt
European Patent Office
Office européen des brevets

(11) EP 1 422 521 A1

(12) EUROPEAN PATENT APPLICATION
published in accordance with Art. 158(3) EPC

(43) Date of publication:
26.05.2004 Bulletin 2004/22

(21) Application number: 02738701.8

(22) Date of filing: 13.06.2002

(51) Int Cl.7: G01N 30/48, B01J 20/22, B01D 15/08, C07C 209/88, C07C 211/27, C07C 33/18, C07C 29/76, C07C 39/14, C07C 37/82, C07B 57/00 // C07M7:00

(86) International application number:
PCT/JP2002/005921

(87) International publication number:
WO 2002/103349 (27.12.2002 Gazette 2002/52)

(84) Designated Contracting States:
AT BE CH CY DE DK ES FI FR GB GR IE IT LI LU MC NL PT SE TR
Designated Extension States:
AL LT LV MK RO SI

(30) Priority: 15.06.2001 JP 2001181501

(71) Applicant: Daicel Chemical Industries, Ltd.
Osaka 590-8501 (JP)

(72) Inventors:
- KASUYA, Natsuki
Saitama-shi, Saitama 330-0835 (JP)
- HABU, Naoto
Utsunomiy a-shi, Tochigi 321-0932 (JP)

(74) Representative: Grünecker, Kinkeldey,
Stockmair & Schwanhäusser Anwaltssozietät
Maximilianstrasse 58
80538 München (DE)

# (54) SEPARATORY AGENT FOR OPTICAL ISOMER

(57) Provided is a separating agent for enantiomeric isomers, that is advantageous from the production standpoints of having high enantiomeric resolution ability inherent in polysaccharide derivatives, solvent resistance, high solubility in a reaction solvent, andgoodfiltrationproperty. Thatis, the separating agent for enantiomeric isomers includes a polysaccharide oligomer derivative derived from a polysaccharide oligomer having a number-average degree of polymerization of from 5 to less than 50, supported on a carrier.

EP 1 422 521 A1

## Description

Art field of the invention

The invention relates to a separating agent for enantiomeric isomers, in particular a separating agent to use for enantiomeric isomers by liquid chromatography and a separating agent for enantiomeric isomers which can optically separate various chiral compounds in analysis of medicines, food, agrochemicals and perfume with a high separation efficiency.

Prior Arts

Many organic compounds have isomers having completely the same physical and chemical properties, examples of physical properties including boiling point, melting point and solubility, but showing a difference in a physiological activity, i.e., enantiomeric isomers. This is attributable to the following. In most cases, proteins and glucides per se constituting a living body of a living organism are composed of only one of the enantiomeric isomers, so that a difference arises in the manner of acting on the other kind of enantiomeric isomers, resulting in difference in the physiological activity. This can be compared to a difference in easiness (difference in physiological activity) of wearing of a glove for left hand (i.e., a living body as an optically active substance) on the right hand and the left hand (respective enantiomeric isomers acting on).

In particular, in the field of pharmaceuticals, in many cases, there are significant differences in medical property and toxicity between two enantiomeric isomers. Therefore, in the Guideline for the Production of Pharmaceuticals, the Ministry of Health, Labour and Welfare describes a policy making a sharp distinction between enantiomeric isomers that "when the drug of interest is a racemic modification, it is desirable to study absorption, distribution, metabolism and excretion of each isomer".

As described above, since the physical properties such as physical and chemical properties, for example, the boiling point, melting point, and solubility of the enantiomeric isomers are quite the same, they cannot be analyzed by the ordinary separation means. Accordingly, studies on the technology of analyzing a wide variety of enantiomeric isomers simply and with high precision have been intensively made. As an analytical technique in response to these requirements, an optical separation method by high performance liquid chromatography (HPLC), in particular an optical separation method by a chiral column for HPLC has been advanced. The chiral column as used herein is an asymmetry identifying agent by itself or a chiral immobilizing phase including an asymmetry identifying agent used as carried on a suitable carrier.

For example, optically active poly(triphenylmethyl methacrylate) (JP 57-150432 A), cellulose, and amylose derivative (Y. Okamoto, M. Kawashima and K. Hatada, J. Am. Chem. Soc., 106, 5337, 1984), and ovomucoid (JP 63-307829 A), which is a protein, have been developed.

Among the many chiral immobilizing phases for HPLC, coating-type optical resolution columns that carry cellulose or an amylose derivative, which is a polysaccharide derivative, on silica gel by physical adsorption are known to have high asymmetry identifying ability for a very wide variety of compounds. In recent years, studies have been advanced on preparative liquid chromatography for an optically active substance on an industrial scale by using a coating-type chiral immobilizing phase for HPLC and a simulated moving bed chromatography (continuously preparative liquid chromatography) in combination (Phram Tech Japan, 12, 43 (1996)).

However, the above separating agent is supported by only physical adsorption. Therefore, a solvent dissolving polysaccharide derivatives cannot be used in a mobile phase or the like, and there is a restriction on the selection of separation conditions. Further, there is a restriction on a solvent dissolving a sample as well, and a sample having a low solubility in a solvent that can be used as a mobile phase has a great disadvantage particularly in preparative chromatography. Furthermore, there is a disadvantage in that the selection of a washing liquid is limited even in the washing of contaminants that strongly adsorb on a separating agent. From those points, a separating agent having polysaccharides supported thereon and also having solvent resistance has been demanded.

To solve this problem, for example, a method of directly chemically bonding polysaccharide derivatives to silica gel is proposed (JP 07-138301A). In this method, when chemically bonding to a carrier such as silica gel, in order to separate polysaccharide-bonded carriers chemically bonded from unreacted polysaccharides by filtration after completion of the reaction, indispensable conditions are that the polysaccharides per se before formation of derivatives thereof are completely dissolved in a reaction solvent, and a dissolution solution has low viscosity for attaining favorable filtration property. However, for polysaccharides that are difficult to dissolve in a solvent and polysaccharides having a degree of polymerization of 50 or more as represented by general cellulose, there is a limit fora dissolution solvent. Even if polysaccharides are dissolved, viscosity becomes very high, and it is difficult to efficiently obtain the objective product by filtration.

Disclosure of the Invention:

Accordingly, a purpose of the present invention is to provide a separating agent for enantiomeric isomers, that is advantageous from the production standpoints of having high enantiomeric resolution ability inherent in polysaccharide derivatives, solvent resistance, high solubility in a reaction solvent, and good filtration property.

The inventors of the present invention have made an intense study in order to solve the above-mentioned problem, and have accordingly achieved the present invention. That is, the present invention relates to a separating agent for enantiomeric isomers characterized in that a polysaccharide oligomer derivative derived from a polysaccharide oligomer having a number-average degree of polymerization of from 5 to less than 50, is supported on a carrier by a chemical bonding.

According to the present invention, there is further provided a method of separating enantiomeric isomers using the separating agent for enantiomeric isomers as described above.

Detailed Description of the Invention:

A polysaccharide oligomer used in the present invention is preferably obtained by depolymerizing a polysaccharide in a phosphoric acid aqueous solution. The polysaccharide used as a raw material can be selected from synthetic polysaccharide, natural polysaccharide, and reformed natural polysaccharide. The polysaccharide preferably has high regulari ty in the binding manner. There are exemplified β-1,4-glucan (cellulose), α-1,4-glucan (amylose, amylopectin), α-1,6-glucan (dextran), β-1,6-glucan (busturan), β-1,3-glucan (for example, cardran, schizophyllan, etc.), α-1,3-glucan, β-1,2-glucan (Crown Gall polysaccharide), β-1,4-galactan, β-1,4-mannan, α-1,6-mannan, β-1,2-fructan (inulin), β-2,6-fructan (levan), β-1,4-xylan, β-1,3-xylan, β-1,4-chitosan, α-1,4-N-acetylchitosan (chitin), pullulan, agarose, alginic acid, and the like. Also, the polysaccharide includes starch containing amylose. Among those, cellulose, amylose, β-1,4-xylan, β-1,4-chitosan, chitin, β-1,4-mannan, inulin, and cardran, which are readily available as the polysaccharide having high purity, are preferred. Cellulose and amylose are particularly preferred. Cellulose is best.

These polysaccharides have a number-average degree of polymerization (an average number of pyranose rings or furanose rings contained in one molecule) of preferably at least 50, and preferably 500 or less in view of ease of handling, though there is no particular limitation in the upper limit thereof.

A phosphoric acid used in depolymerization reaction of the polysaccharide has a phosphoric acid concentration of preferably 75 to 93% by weight, particularly preferably 82 to 87% by weight. Reaction temperature and reaction time of this depolymerization reaction are not particularly limited in the present invention. Reaction proceeds even at room temperature, and by leaving to stand at room temperature for an appropriate period of time, the objective polysaccharide oligomer is obtained. Further, the reaction can be promoted at an elevated temperature. The reaction temperature is selected from a range of generally 20 to 90°C, and preferably 30 to 70°C.

The polysaccharide oligomer used in the present invention has a number-average degree of polymerization (average number of pyranose or furanose rings per molecule) of 5 or more, and preferably 7 or more, with the upper limit being less than 50. If it exceeds 50, there is limit on a dissolution solvent, and even if the polysaccharide oligomer is dissolved, the viscosity becomes very high, and the objective product cannot be efficiently obtained by filtration.

Examples of the polysaccharide oligomer derivative used in this reaction include compounds obtained by combining part of hydroxyl groups of the polysaccharide oligomer with a compound having a functional group capable of reacting with the hydroxy group by way of ester-bonding, urethane-bonding, ether-bonding or the like according to a conventional method and deriving it. The polysaccharide oligomer derivatives particularly preferably used here include ester derivatives and carbamate derivatives. The compound having a functional group capable of reacting with hydroxyl group can be any of isocyanic acid derivatives, carboxylic acids, esters, acid halides, acid amides, halides, epoxides, aldehydes, alcohols and other compounds having leaving groups. Examples of those compounds include aliphatic, alicyclic, aromatic, and heteroaromatic compounds.

The polysaccharide oligomer derivative used in the present invention are that when a weight-average molecular weight is represented by DPw and a number-average molecular weight is represented by DPn, distribution polymerization degree is preferably $DPw/DPn<3.0$, and more preferably $DPw/DPn<2.5$.

The carrier used in the present invention includes organic porous carriers and inorganic porous carriers, preferably inorganic porous carriers. Appropriate examples of the organic porous carriers include polymer substances comprising polystyrene, polyacrylamide, polyacrylate or the like. Appropriate examples of the inorganic porous carriers include silica, alumina, magnesia, glass, kaolin, titanium oxide, silicates, hydroxyapatites, or the like. Particularly preferable carrier is silica gel having a particle diameter of 0.1 μm to 10 mm, preferably 1 μm to 300 μm, and an average pore diameter of 10 to 100 μm, preferably 50 to 50, 000 Å.

It is desirable that surface treatment with a silane treating agent or the like is applied to a carrier surface in order to remove the influence of residual silanol. A compound having an amino group is used as a surface treating agent. In particular, a compound having a primary amine is preferable. The surface treating agent can optionally use

any of a silane coupling agent, commercially available, and a product synthesized so as to have amine, and the like. Examples thereof include 3-aminopropyltriethoxysilane and 3-(2-aminoethylpropyl)tri-methoxysilane.

The separating agent, a polysaccharide oligomer derivative supported on a carrier according to the present invention, is the agent in which the polysaccharide oligomer derivative may be applied to a carrier and supported thereon by physical adsorption, or may further be strongly fixed to the carrier by forming additional chemical bonding by a chemical bond between the carrier and the polysaccharide oligomer derivative applied, a chemical bond between mutual polysaccharide oligomer derivatives on the carrier, a chemical bond using a third component, a light irradiation to the polysaccharide oligomer derivative on the carrier, an irradiation with radiation such as γ-rays, an irradiation with electromagnetic wave such as microwave, a radical reaction, or the like. Further, the polysacchararaide oligomer derivative as an asymmetry identification agent and a polymer compound, which is not optically active, may simultaneously be supported. Of those, the polysaccharide oligomer derivative chemically bonded to a carrier is preferable.

In producing the separating agent of the present invention, the polysaccharide oligomer may be converted into its derivative, and the derivative may be chemically bonded to the above carrier, or the polysaccharide oligomer maybe chemically bonded to the carrier, and then converted into its derivative. However, the agent in which chemical bonding is formed by a chemical reaction between a reducing terminal site of the polysaccharide oligomer or its derivative and an amino group on the carrier is preferable.

The separating agent for enantiomeric isomers of the present invention is generally used in chromatography such as gas chromatography, liquid chromatography, thin layer chromatography, supercritical chromatography or capillary electrophoresis, and enantiomeric resolution by membrane separation method or the like. It is particularly preferable to be used as a chiral immobilizing phase for liquid chromatography. More particularly, it is preferable to be used as a chiral immobilizing phase for the purpose of analysis or an immobilizing phase for continuous liquid preparative chromatography represented by a simulated moving bed method.

Effect of the Invention:

According to the present invention, there can be provided a separating agent for enantiomeric isomers advantageous from production standpoints of being capable of greatly improving solubility of cellulose in a reaction agent, having high enantiomeric resolution ability inherent in cellulose derivatives, having solvent resistance and having good filtration property.

Examples

Hereinafter, the present invention is described in more detail with reference to the examples, but the invention is not limited to those examples.

Synthesis Example 1: Preparation of monodisperse cellulose oligomer

50 g of CF-11 (cellulose, produced by Whatman P1c), 36.5 ml of water, and 935 ml of 85% phosphoric acid were placed in a flask, and the resultant mixture was stirred with a glass rod according to the method of Isogai et al. (Mokuzai Gakkaishi, Vol.37, No.4, 339-344 (1991)), and then left to stand for 6 weeks to depolymerize the cellulose. Thereafter, 3 liters of water was added to this cellulose oligomer aqueous solution to obtain a white precipitate. This solution was suspended inwater, and water and cellulose oligomer were separated with a centrifuge (5000 rpm for 5 min). One liter of water was further added to the precipitate, and centrifugation was repeated three times. The precipitate was neutralized with dilute NaOH aqueous solution, 1 liter of water was further used, and centrifugation was repeated two times. The precipitate was left to stand overnight at 40°C to obtain low molecular weight cellulose oligomer.

The obtained cellulose oligomer was converted into a phenyl isocyanate, and a degree of polymerization was analyzed by a polystyrene conversion method using standard polystyrene with size exclusion chromatography (column: TSK gel G25000HxL, G40000HxL, produced by Tosoh Corporation). As a result, it was found that the degree of polymerization of CF-11 as a raw material is 136, and the distribution of polymerization degree (DPw/DPn) of CF-11 converted into a phenyl isocyanate (cellulose triphenylcarbamate) is 11.4, whereas the degree of polymerization of the monodisperse cellulose oligomer obtained is 15 and the distribution of polymerization degree (DPw/DPn) of cellulose oligomer converted into a phenyl isocyanate (cellulose oligomer triphenylcarbamate) is 2.0.

Example 1

(1) Silica gel surface treatment

Porous silica gel (particle diameter 7 μm, pore diameter 128 Å) was reacted with 3 - aminopropyltriethoxysilane

in the conventional manner to obtain aminopropylsilane-treated (APS treated) silica gel having a pore diameter of 128 Å.

(2) Chemical bonding of pentadecamer cellulose oligomer to silica gel

4.0 g of dry lithium chloride was dissolved in 50 ml of anhydrous N,N-dimethylacetamide (DMAc). 1.0 g of pentadecamer cellulose oligomer obtained in (1) was dissolved in 29.4 ml of the above DMAc/LiCl solution, and dissolved by heating at 80°C for 12 hours. 40 µl of acetic acid, 6.8 ml of dimethylsulfoxide (DMSO), 0.2 g of boron cyanosodium hydride and APS treated silica gel (pore diameter 128 Å) obtained in Synthesis Example 1 were added thereto. The resultant mixture was stirred by heating at 80°C for 48 hours, allowed to stand to cool and then filtered through a glass filter to obtain cellulose oligomer-bonded silica gel. The filtration in this case was conducted with an aspirator, and the whole amount of the reaction solution could be filtered in about 30 minutes. The cellulose oligomer-bonded silica gel filtered off was washed with 25 ml of DMAc/LiCl solution, 25mlofacetoneand25mlofhexane, successively, three times, and dried in vacuo at 40°C overnight.

(3) Conversion of cellulose oligomer chemically bonded to silica gel into phenyl carbamate derivative

5.0 g of cellulose oligomer-bonded silica gel prepared in (2) was dried, and 13.3 ml of anhydrous DMAc/LiCl solution, 5.0 ml of anhydrous pyridine, and 3.3 ml of phenyl isocyanate were added thereto. Reaction was conducted at 90°C for 12 hours while stirring. After the reaction, about 10 ml of methanol was added to the reaction system, and the resultant mixture was allowed to stand to cool and then filtered through a glass filter to obtain a cellulose oligomer chemically bonded to silica gel converted into a derivative. In this case, filtration was conducted well. The cellulose oligomer chemically bonded to silica gel which was filtered off and converted into a derivative was washed with 25 ml of DMAc, 25 ml of acetone, and 25 ml of hexane, successively, three times, and dried in vacuo at 40°C overnight. By this procedure, cellulose oligomer phenyl carbamate derivative-bonded silica gel having a pore diameter of 128 Å was obtained.

Example 2

Cellulose oligomer phenyl carbamate derivative-bonded silica gel having a pore diameter of 660 Å was obtained in the same manner as in Example 1, except for using porous silica gel having a pore diameter of 660 Å and particle diameter of 7 µm in place of porous silica gel having a pore diameter of 128 Å.

Example 3

(1) Silica gel surface treatment

APS treated silica gel having a pore diameter of 128 Å was obtained in the same manner as in Example 1 (1).

(2) Chemical bonding of pentadecamer cellulose oligomer to silica gel

Pentadecamer cellulose oligomer-bonded silica gel was obtained in the same manner as in (2) in Example 1.

(3) Conversion of cellulose oligomer chemically bonded to silica gel into 3,5-dichlorophenylcarbamate derivative

5.0 g of cellulose oligomer-bonded silica gel prepared in (2) was dried, and 13.3 ml of anhydrous DMAc/LiCl solution, 5.0 ml of anhydrous pyridine, and 3.3 ml of 3,5-dichlorophenyl isocyanate were added thereto. Reaction was conducted at 90°C for 12 hours under stirring. After the reaction, about 10 ml of methanol was added to the reaction system, and the resultant mixture was allowed to stand to cool and then filtered through a glass filter to obtain a cellulose oligomer derivative-bonded silica gel. In this case, filtration was conducted well. The cellulose oligomer derivative-bonded silica gel which was filtered off was washed with 25 ml of DMAc, 25 ml of acetone, and 25 ml of hexane, successively, three times, and dried in vacuo at 40°C overnight. By this procedure, cellulose oligomer 3,5-dichlorophenylcarbamate derivative-bonded silica gel having a pore diameter of 128 Å was obtained.

Elemental analysis values of cellulose oligomer phenyl carbamate derivative-bonded silica gels obtained in Examples 1 and 2, and each silica gel and cellulose oligomer-bonded silica gel that are raw materials, and elemental analysis value of cellulose oligomer-3,5-dichlorophenylcarbamate derivative-bonded silica gel obtained in Example 3 are shown in Table 1.

Table 1

| | | Carbon content (C%) | Nitrogen content (N%) |
|---|---|---|---|
| Ex. 1 | Silica gel (128 Å) | 4.22 | 1.39 |
| | Cellulose oligomer-bonded gel (128 Å) | 6.92 | 0.72 |
| | Cellulose oligomer phenyl carbamate derivative-bonded silica gel (128 Å) | 16.71 | 2.92 |
| Ex. 2 | Silica gel (660 Å) | 0.97 | 0.40 |
| | Cellulose oligomer-bonded silica gel (660 Å) | 1.99 | 0.26 |
| | Cellulose oligomer phenyl carbamate derivative-bonded silica gel (660 Å) | 4.18 | 0.76 |
| Ex. 3 | Cellulose oligomer 3,5-dechlorophenylcarbamate derivative-bonded silica gel (128 Å) | 11.67 | 2.35 |

Comparative Example 1

(1) Silica gel surface treatment

Porous silica gel (particle diameter 7 μm, pore diameter 660 Å) was reacted with 3-aminopropyltriethoxysilane in the conventional manner to obtain aminopropylsilane-treated (APS treated) silica gel.

(2) Chemical bonding of cellulose to silica gel

4.0 g of dry lithium chloride was dissolved in 50 ml of anhydrous N,N-dimethylacetamide (DMAc). 1.0 g of cellulose, CF-11 (degree of polymerization 136, distribution of polymerization degree 11.4), a product of Whatman Plc was dissolved in 29.4 ml of the above-mentioned DMAc/LiCl solution and dissolved by heating at 80°C for 12 hours. 40 μl of acetic acid, 6.8 ml of dimethylsulfoxide (DMSO), 0.2 g of boron cyanosodium hydride, and 4.0 g of APS treated silicagelof theabove-mentioned (1) were added to the above solution. The resultant solution was stirred under heating at 80°C for 48 hours, and allowed to stand to cool. An attempt was made to obtain cellulose-bonded silica gel by filtration with a glass filter, but about 23 ml of the reaction solution had very high viscosity, so that it took more than 10 hours to filtrate the solution with an aspirator. Therefore, it was abandoned to filter off the objective product.

From this result, it was concluded that the preparation of a cellulose derivative-bonded separating agent using cellulose not subjected to molecular weight reduction as a raw material is difficult.

Example 4

(1) Silica gel surface treatment

Using a porous silica gel (particle diameter 7 μm, pore diameter 1000 Å), APS treated silica gel having a pore diameter of 1000 Å was obtained in the same manner as in (1) in Example 1.

(2) Synthesis of cellulose triphenylcarbamate

5.0 g of cellulose pentadecamer prepared in Synthesis Example 1 was dispersed in 80 ml of dry pyridine in a nitrogen atmosphere, and 22 g of phenyl isocyanate was added thereto to conduct reaction for 30 hours under reflux. After completion of the reaction, the reaction mixture was allowed to cool down to room temperature and poured in excess methanol. Solid precipitates were filtered with a glass filter and dried in vacuum at 60°C to obtain 13.6 g (yield: 85%) of a slightly yellow cellulose triphenylcarbamate.

(3) Preparation of filler having cellulose triphenylcarbamate supported thereon by physical adsorption

1.0 g of cellulose triphenylcarbamate prepared in the above-mentioned (2) was dissolved in tetrahydrofuran, and the resultant solution was uniformly applied to APS treated silica gel of the above-mentioned (1) to obtain a filler.

Comparative Example 2

(1) Silica gel surface treatment

APS treated silica gel having a pore diameter of 1000 Å was obtained in the same manner as in Example 4 (1).

(2) Synthesis of cellobiose tris(3,5-dimethylphenylcarbamate)

1.0 g of cellobiose was dispersed in 10 ml of dry pyridine in a nitrogen atmosphere, and 5.4 g of 3,5-dimethylphenyl isocyanate was added thereto to conduct reaction at 80°C for 24 hours. After completion of the reaction, the reaction mixture was allowed to stand to cool down to room temperature, and pyridine and excess isocyanate were distilled off under reduced pressure to obtain a solid product. The crude product obtained was thoroughly washed with hexane, and then dissolved in tetrahydrofuran. Insoluble matter was removed by filtration, and soluble matter was poured in methanol/water=4/1 to obtain the objective cellobiose tris(3,5-dimethylphenylcarbamate). Solid precipitated was filtered off with a glass filter, and dried in vacuo at 60°C to obtain 1.67 g (yield: 45%) of slightly yellow cellobiose tris(3,5-dimethylphenylcarbamate).

(3) Preparation of filler having cellobiose tris(3,5-dimethylphenylcarbamate) supported thereon by physical adsorption

1.0 g of cellobiose tris(3,5-dimethylphenylcarbamate) prepared in the above-mentioned (2) was dissolved in tetrahydrofuran, and the resultant solution was uniformly applied to APS treated silica gel of the above-mentioned (1) to obtain a filler.

Comparative Example 3

(1) Silica gel surface treatment

APS treated silica gel having a pore diameter of 1000 Å was obtained in the same manner as in (1) in Example 4.

(2) Synthesis of cellotetraose tris(3,5-dimethylphenylcarbamate)

1.86 g (yield: 50%) of cellotetraose tris(3,5-dimethylphenylcarbamate) was obtained in the same manner as in Comparative Example 2 (2), except for using cellotetraose in place of cellobiose.

(3) Preparation of filler having cellotetraose tris(3,5-dimethylphenylcarbamate) supported thereon by physical adsorption

1.0 g of cellotetraose tris(3,5-dimethylphenylcarbamate) prepared in the above-mentioned (2) was dissolved in tetrahydrofuran, and the resultant solution was uniformly applied to APS treated silica gel of the above-mentioned (1) to obtain a filler.

Application Example 1

Cellulose oligomer derivative-bonded silica gels prepared in Examples 1 to 3 were, used as a filler, packed in a stainless steel-made column having a length of 25 cm and an inner diameter of 0.46 cm by a slurry packing method to prepare three kinds of separation columns for enantiomeric isomers. Using the columns, enantiomeric resolution of racemic modification compounds 1 to 6 represented by the following formulae was conducted by a liquid chromatography under the following analysis conditions. The results are shown in Table 2.

<Analysis Conditions>

Mobile phase: Racemic modification compound 1 is n-hexane/2-propanol/dimethylamine=90/10/0.5 (v/v/v), racemic modification compounds 2 to 5 are n-hexane/2-propanol=90/10 (v/v), and racemic modification compound 6 is n-hexane/2-propanol/trifluoroacetic acid=90/10/0.5 (v/v/v).

Flow rate: 0.5 ml/min
Temperature: 25°C

Detection: 254 nm

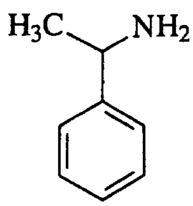

Racemic Compound 1

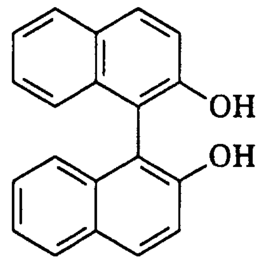

Racemic Compound 2

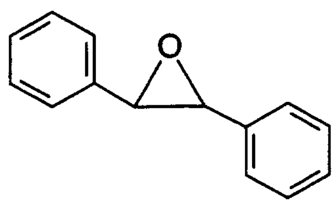

Racemic Compound 3

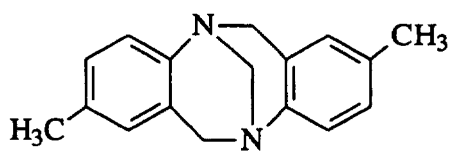

Racemic Compound 4

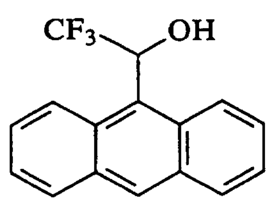

Racemic Compound 5

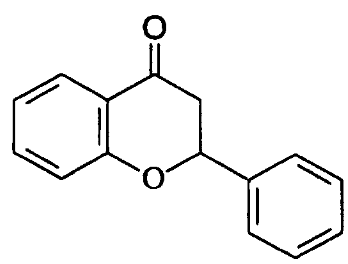

Racemic Compound 6

In Table 2, holding coefficient ($k_1$') of a relatively weakly held enantiomeric isomer, holding coefficient ($k_2$') of a relatively strongly held enantiomeric isomer and separation factor ($\alpha$) are defined by the following equations.

$$k_1' = \frac{(t_1 - t_2)}{t_0}$$

$$k_2' = \frac{(t_2 - t_2)}{t_0}$$

$$\alpha = \frac{k_2'}{k_1'}$$

(in the equations, $t_1$ is an elution time of a relatively weakly held enantiomeric isomer, $t_2$ is an elution time of a relatively strongly held enantiomeric isomer, and $t_0$ is an elution time of tri-tert-butylbenzene.)

Table 2

| Filler | Racemic compound | $t_1$ | $t_2$ | $k_1'$ | $k_2'$ | α value |
|---|---|---|---|---|---|---|
| Separating agent of Ex. 1 | 4 | 14.10 | 14.68 | 0.57 | 0.63 | 1.11 |
| | 5 | 25.87 | 28.15 | 1.87 | 2.13 | 1.14 |
| Separating agent of Ex. 2 | 5 | 8.43 | 8.66 | 0.26 | 0.29 | 1.14 |
| Separating agent of Ex. 3 | 1 | 9.86 | 11.96 | 0.74 | 1.12 | 1.50 |
| | 2 | 21.59 | 22.43 | 2.82 | 2.97 | 1.05 |
| | 3 | 9.75 | 9.91 | 0.73 | 0.75 | 1.04 |
| | 4 | 8.41 | 9.03 | 0.49 | 0.60 | 1.23 |
| | 5 | 9.50 | 9.88 | 0.68 | 0.75 | 1.10 |
| | 6 | 15.11 | 15.50 | 1.67 | 1.74 | 1.04 |

Application Example 2

The fillers prepared in Examples 4 and Comparative Examples 2 and 3 were packed in a stainless steel-made column having a length of 25 cm and an inner diameter of 0.46 cm by a slurry packing method to prepare three kinds of separation columns for enantiomeric isomers. Using these columns, enantiomeric resolution of racemic modification compounds 3 to 5 represented by the above formulae was conducted by a liquid chromatography under the following analysis conditions. The results are shown in Table 3. Note that $t_1$, $t_2$, $k_1'$, $k_2'$, and α represent the same meanings as those in Table 2.

<Analysis Conditions>

In Example 4, the following conditions are set.

Mobile phase: n-hexane/2-propanol=90/10 (v/v)
Flow rate: 1.0 ml/min
Temperature: 25°C
Detection: 254 nm

In Comparative Examples 2 and 3, the following conditions are set.

Mobile phase: n-hexane
Flow rate: 0.5 ml/min
Temperature: 25°C
Detection: 254 nm

Table 3

| Filler | Racemic compound | $t_1$ | $t_2$ | $k_1'$ | $k_2'$ | α value |
|---|---|---|---|---|---|---|
| Example 1 | 3 | 5.09 | 5.58 | 0.54 | 0.69 | 1.27 |
| | 4 | 6.34 | 6.76 | 1.02 | 1.15 | 1.13 |
| | 5 | 7.92 | 9.62 | 1.52 | 2.06 | 1.36 |

Table 3 (continued)

| Filler | Racemic compound | $t_1$ | $t_2$ | $k_1'$ | $k_2'$ | α value |
|---|---|---|---|---|---|---|
| Comparative Example 2 | 3 | 14.5 | 17.6 | 1.38 | 1.89 | 1.37 |
| | 4 | 7.69 | - | 0.26 | - | 1.00 |
| | 5 | 7.63 | - | 0.25 | - | 1.00 |
| Comparative Example 3 | 3 | 62.3 | - | 9.38 | - | 1.00 |
| | 4 | 15.2 | 17.2 | 1.54 | 1.86 | 1.21 |
| | 5 | 18.8 | 20.5 | 2.13 | 2.41 | 1.13 |

## Claims

1. A separating agent for enantiomeric isomers, comprising a polysaccharide oligomer derivative derived from a polysaccharide oligomer having a number-average degree of polymerization of from 5 to less than 50, supported on a carrier.

2. The separating agent for enantiomeric isomers as claimed in claim 1, wherein a distribution of polymerization degree of the polysaccharide oligomer derivative is DPw/DPn<3.0, DPw representing a weight-average molecular weight, DPn representing a number-average molecular weight.

3. The separating agent for enantiomeric isomers as claimed in claim 1 or 2, wherein the polysaccharide oligomer is obtained by depolymerizing a polysaccharide in a phosphoric acid aqueous solution.

4. The separating agent for enantiomeric isomers as claimed in any one of claims 1 to 3, wherein the polysaccharide oligomer derivative is at least one selected from the group consisting of ester derivatives and carbamate derivatives.

5. The separating agent for enantiomeric isomers as claimed in any one of claims 1 to 4, wherein the polysaccharide oligomer derivative is supported on the carrier by chemical bonding.

6. The separating agent for enantiomeric isomers as claimed in claim 5, wherein the chemical bonding is formed by a chemical reaction between a reducing terminal site of the polysaccharide oligomer or its derivative and an amino group on the carrier.

7. The separating agent for enantiomeric isomers as claimed in any one of claims 1 to 6, wherein the polysaccharide is a cellulose.

8. The separating agent for enantiomeric isomers as claimed in any one of claims 1 to 7, wherein the separating agent is used as a chiral immobilizing phase for a liquid chromatography.

9. The separating agent for enantiomeric isomers as claimed in claim 8, wherein the chiral immobilizing phase for a liquid chromatography is used for the purpose of analysis.

10. The separating agent for enantiomeric isomers as claimed in claim 8, wherein the chiral immobilizing phase for a liquid chromatography is an immobilizing phase for a continuous liquid preparative chromatography.

11. A method of separating enantiomeric isomers using the separating agent for enantiomeric isomers as claimed in any one of claims 1 to 10.

## INTERNATIONAL SEARCH REPORT

International application No.
PCT/JP02/05921

**A. CLASSIFICATION OF SUBJECT MATTER**

Int.Cl[7] G01N30/48, B01J20/22, B01D15/08, C07C209/88, C07C211/27, C07C33/18, C07C29/76, C07C39/14, C07C37/82, C07B57/00, C07M7:00

According to International Patent Classification (IPC) or to both national classification and IPC

**B. FIELDS SEARCHED**

Minimum documentation searched (classification system followed by classification symbols)

Int.Cl[7] G01N30/48, B01J20/22, B01D15/08, C07C209/88, C07C211/27, C07C33/18, C07C29/76, C07C39/14, C07C37/82, C07B57/00, C07M7:00

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2002 |
| Kokai Jitsuyo Shinan Koho | 1971-2002 | Jitsuyo Shinan Toroku Koho | 1996-2002 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | JP 6-206895 A (Nakano Vinegar Co., Ltd.), 26 July, 1994 (26.07.94), (Family: none) | 1,4-6,8,9,11<br>2,4-11<br>3 |
| X<br>Y<br>A | JP 6-322001 A (Nakano Vinegar Co., Ltd.), 22 November, 1994 (22.11.94), (Family: none) | 1,4-6,8,9,11<br>2,4-11<br>3 |
| Y<br>A | JP 8-113541 A (Daicel Chemical Industries, Ltd.), 07 May, 1996 (07.05.96), (Family: none) | 2,4-11<br>3 |
| Y | JP 8-29404 A (Daicel Chemical Industries, Ltd.), 02 February, 1996 (02.02.96), (Family: none) | 10,11 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 July, 2002 (15.07.02) | 30 July, 2002 (30.07.02) |
| Name and mailing address of the ISA/<br>Japanese Patent Office | Authorized officer |
| Facsimile No. | Telephone No. |

Form PCT/ISA/210 (second sheet) (July 1998)